# EUROPEAN PATENT APPLICATION

(11) **EP 4 732 856 A1**
(43) Date of publication of application: **29.04.2026**
(21) Application number: 24825973.1
(22) Date of filing: 20.06.2024
(51) Int. Cl.: A61L 27/12, A61L 27/38, A61L 27/40

(54) **COMPLEX FOR BONE REGENERATION OR AUGMENTATION, AND METHOD FOR PRODUCING SAME**

(30) Priority: 21.06.2023 JP 2023102016
(71) Applicant: Kagoshima University, Kagoshima-shi, Kagoshima 890-8580 (JP)
(72) Inventor: NISHIMURA, Masahiro, Kagoshima-shi, Kagoshima 890-8580 (JP); SUEHIRO, Fumio, Kagoshima-shi, Kagoshima 890-8580 (JP); KOMABASHIRI, Naohiro, Kagoshima-shi, Kagoshima 890-8580 (JP)
(74) Representative: Weickmann & Weickmann PartmbB
(86) International application number: PCT/JP2024/022387
(87) International publication number: WO 2024/262572

(57) **Abstract**

The present invention provides a complex for bone regeneration or augmentation, containing bone substitute material covered with a layered cell sheet containing osteogenic cells.

## Description

### [Technical Field]

The present invention relates to a complex for bone regeneration or augmentation and a production method thereof. More particularly, the present invention relates to a complex for bone regeneration or augmentation which is obtained by covering bone substitute material with a layered cell sheet containing osteogenic cells, and a production method thereof.

### [Background Art]

In the orthopedic field and dental field, intensive studies are underway to construct a method for regenerating or augmenting bones lost due to accidents, illnesses, or congenital abnormalities (Patent Literatures 1 - 3).

Autologous bone grafting is the gold standard in the conventional bone regeneration treatments. However, because autologous bone grafting requires high techniques and complicated equipment to harvest autologous bone, it is extremely difficult to perform it safely and conveniently. It is a treatment method requiring improvement because it is highly invasive and imposes a great burden on patients. Therefore, there is a strong need for the construction of alternative methods to autologous bone grafting.

As alternative methods to autologous bone grafting, for example, a method using biocompatible materials such as hydroxyapatite as a bone substitute material, a method using osteogenic cells (mesenchymal stem cells, osteoblasts, etc.) involved in bone formation in the body, and a method using growth factors such as basic fibroblast growth factor (bFGF) and bone morphogenetic protein (BMP) are under investigation.

However, these alternative methods also have problems. For example, when using biocompatible materials as bone substitute materials, bone regeneration takes a relatively long time because the bone substitute materials implanted into the body does not actively interact with biological tissues. Furthermore, such bone substitute materials are generally very small granular materials (about 0.15 to 2 mm), and pose difficulty in operability. Furthermore, the methods using osteogenic cells require the proliferation of autologous cells, and the preparation of the required number of cells takes a lot of time and money. Furthermore, the methods using growth factors also have problems in that bone regeneration cannot be precisely controlled, production costs are high, and the results may vary depending on the patient's health conditions.

In addition, a problem common to these methods is the difficulty in achieving horizontal/vertical bone augmentation.

Specifically, in bone regeneration and augmentation, it is relatively easy to regenerate or augment bone defects resulting from bone marrow cavities caused by tooth extraction socket, fracture, and the like. However, it is extremely difficult for conventional bone augmentation methods to increase bone width (i.e., horizontal bone augmentation) or bone height (i.e., vertical bone augmentation) in extensive bone defect sites caused by accidents, illnesses, congenital abnormalities or the like.

Therefore, there is a strong demand for the establishment of a novel bone regeneration/augmentation method that can solve such various problems in conventional techniques.

### [Citation List]

### [Patent Literature]

[Patent Literature 1]
   WO2014/115562
[Patent Literature 2]
   WO2014/017147
[Patent Literature 3]
   WO2020/226043

### [Summary of Invention]

### [Technical Problem]

The present invention aims to provide a novel bone regeneration/augmentation tool that enables horizontal/vertical bone regeneration or augmentation and has good operability. The present invention also provides a method for producing the tool easily and efficiently.

### [Solution to Problem]

The present inventors have conducted intensive studies of the above-mentioned problems and found that a complex obtained by covering bone substitute material with a layered cell sheet containing mesenchymal stem cells (1) enables horizontal and vertical bone augmentation by implanting a complex of an appropriate size for the size of the bone defect, (2) when implanted into a living body, new bone can be formed not only on the host bone side but also in the center of the complex, (3) when implanted into a living body, bone marrow-like tissue is formed in addition to bone formation, thus reproducing a structure similar to bone tissue present in the living body, and (4) when a complex is produced using only two elements, bone substitute material and a layered cell sheet, it can be produced by very simple steps, and as a result, a complex of uniform quality can be produced efficiently and easily. Furthermore, they have found that, particularly when using granular bone substitute materials, (5) operability is significantly improved by covering the fine particulate bone substitute materials with a layered cell sheet to form a substantially spherical complex, (6) a complex for bone regeneration/augmentation of a desired size can be prepared by adjusting the amount of bone substitute materials and the area of the layered cell sheet, and (7) because the complex is substantially spherical, the orientation of the complex is not restricted during implantation, and the like. Based on such findings, the present inventors have conducted further studies and completed the present invention.

That is, the present invention provides the following.
[1] A complex for bone regeneration or augmentation, comprising bone substitute material covered with a layered cell sheet containing osteogenic cells.
[2] The complex of [1], wherein the bone substitute material is at least one selected from the group consisting of hydroxyapatite, β-tricalcium phosphate, and carbonate apatite.
[3] The complex of [1] or [2], wherein the osteogenic cell is at least one selected from the group consisting of a mesenchymal stem cell, an osteoblast, and a bone marrow-derived stromal cell.
[4] A method for producing a complex for bone regeneration or augmentation, comprising the following step:
   a step of covering bone substitute material with a layered cell sheet comprising osteogenic cells.
[5] The production method of [4], comprising a step of preparing the layered cell sheet comprising osteogenic cells before the step of covering bone substitute material with a layered cell sheet comprising osteogenic cells.
[6] The production method of [4] or [5], wherein the bone substitute material is at least one selected from the group consisting of hydroxyapatite, β-tricalcium phosphate, and carbonate apatite.
[7] The production method of any of [4] to [6], wherein the osteogenic cell is at least one selected from the group consisting of a mesenchymal stem cell, an osteoblast, and a bone marrow-derived stromal cell.
[8] A kit for producing a complex for bone regeneration or augmentation, comprising bone substitute material and a layered cell sheet comprising osteogenic cells.
[9] The kit of [8], wherein the bone substitute material is at least one selected from the group consisting of hydroxyapatite, β-tricalcium phosphate, and carbonate apatite.
[10] The kit of [8] or [9], wherein the osteogenic cell is at least one selected from the group consisting of a mesenchymal stem cell, an osteoblast, and a bone marrow-derived stromal cell.
[11] A layered cell sheet comprising osteogenic cells, which is for use in producing a complex for bone regeneration or augmentation by combining with bone substitute material.
[12] The layered cell sheet of [11], wherein the bone substitute material is at least one selected from the group consisting of hydroxyapatite, β-tricalcium phosphate, and carbonate apatite.
[13] The layered cell sheet of [11] or [12], wherein the osteogenic cell is at least one selected from the group consisting of a mesenchymal stem cell, an osteoblast, and a bone marrow-derived stromal cell.
[14] A bone substitute material, which is for use in producing a complex for bone regeneration or augmentation by combining with a layered cell sheet comprising osteogenic cells.
[15] The bone substitute material of [14], wherein the bone substitute material is at least one selected from the group consisting of hydroxyapatite, β-tricalcium phosphate, and carbonate apatite.
[16] The bone substitute material of [14] or [15], wherein the osteogenic cell is at least one selected from the group consisting of a mesenchymal stem cell, an osteoblast, and a bone marrow-derived stromal cell.

### [Advantageous Effects of Invention]

According to the present invention, it is possible to treat a wide range of bone defects requiring horizontal/vertical bone regeneration and/or bone augmentation. According to the present invention, moreover, it is possible to efficiently prepare a novel complex for bone regeneration or augmentation that enables such treatment. In particular, while it has been reported that layered implanted cells cannot remain in the local area and either migrate to other sites or die, in Examples of the present invention, it was shown that the implanted cells not only survived 12 weeks after implantation, but also differentiated into osteocytes. This is considered to be because an environment permitting cell survival is maintained by the layered cells and extracellular matrix, and cytokines secreted by the surviving cells exhibits the paracrine/autocrine effect.

### [Brief Description of Drawings]

[Fig. 1]

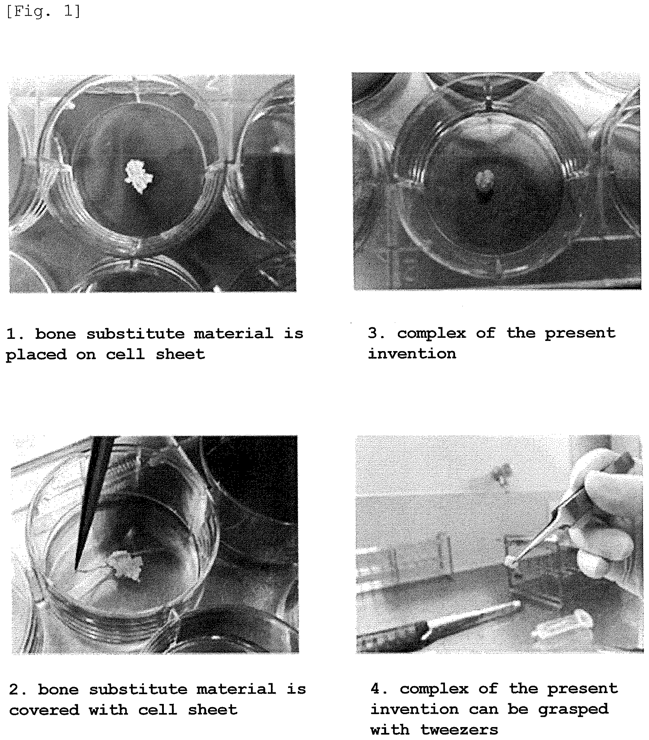

   Fig. 1 is a diagram showing one embodiment of the complex of the present invention.
[Fig. 2]

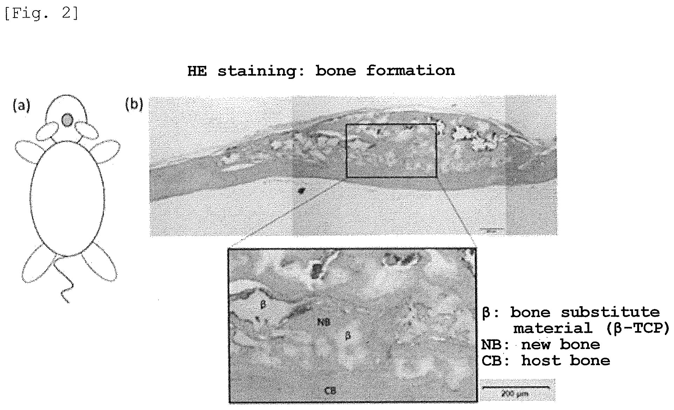

   Fig. 2 is a diagram showing the results of implanting a complex of the present invention prepared using rat femur-derived bone marrow mesenchymal stem cells into the parietal region of a rat. (a) shows the implantation site of the complex of the present invention. (b) shows the results of HE staining of the implantation site 12 weeks after implantation.
[Fig. 3]

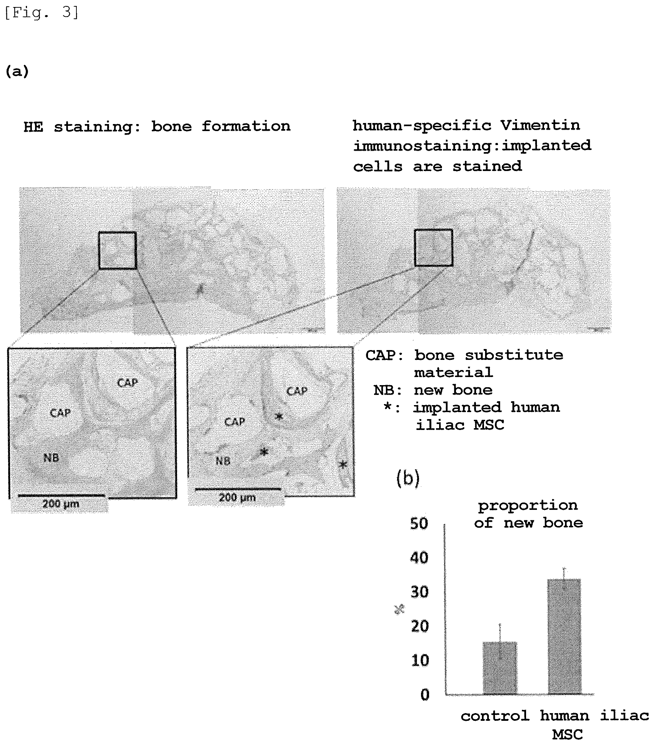

   Fig. 3 is a diagram showing the results of implanting a complex of the present invention prepared using human iliac bone marrow mesenchymal stem cells into an immunodeficient mouse. (a) shows the results of HE staining and immunostaining for human-specific Vimentin of the implantation site 12 weeks after implantation. (b) shows the proportion of new bone formed in the control and the complex of the present invention (human iliac MSCs) .
[Fig. 4]

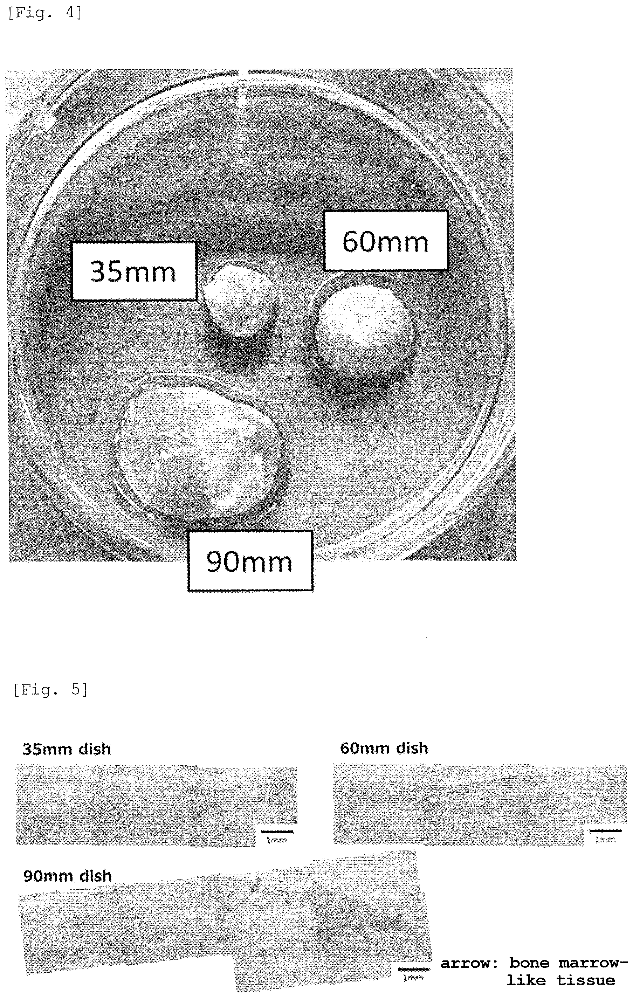

   Fig. 4 is a diagram showing the appearance of three complexes with different sizes of the present invention prepared in a 35 mm dish, a 60 mm dish, and a 90 mm dish.
[Fig. 5]
   Fig. 5 is a diagram showing the results of HE staining of the implant site 12 weeks after implantation, when complexes with different sizes of the present invention were implanted into the parietal region of a rat.
[Fig. 6]

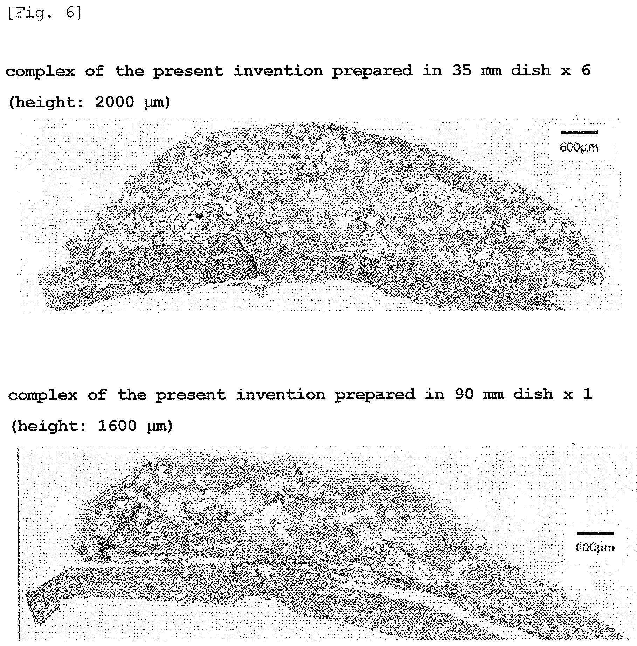

   Fig. 6 is a diagram showing the results when complexes with different sizes of the present invention were implanted into rats under conditions where the number of cells present on the surface of the complex was standardized.

### [Description of Embodiments]

The present invention is described in detail below.

### 1. Complex for bone regeneration or augmentation

The present invention provides a complex for bone regeneration or augmentation, containing bone substitute material covered with a layered cell sheet containing osteogenic cells (hereinafter sometimes referred to as "the complex of the present invention").

In the present specification, the "bone substitute material" refers to a biocompatible and osteoconductive material used for bone regeneration and augmentation in the orthopedic field and dental field. The "bone substitute material" includes autologous bone or processed materials derived therefrom, allogeneic bone or processed materials derived therefrom, xenogenic bone or processed materials derived therefrom, and artificial bone. In the present invention, these bone substitute materials may be used alone or in combination of two or more types. In one preferred embodiment of the present invention, the bone substitute material may be artificial bone. Examples of artificial bones that can be used in the present invention include, but are not limited to, hydroxyapatite (HA), β-tricalcium phosphate (β-TCP), and carbonate apatite (CAP), and the like. The bone substitute material that can be used in the complex of the present invention can be prepared by methods known per se, or commercially available products may be used. Examples of the commercially available bone substitute material include HA such as "Apaceram" (HOYA Technosurgical Corporation), "Neobone" (Kentec Co., Ltd.), and "Calcitite" (Hakuho Co., Ltd.), β-TCP such as "OSPHERION (registered trademark)" (Olympus Corporation), "Cerasorb (registered trademark)" (Ginvi Japan LLC), and "Superpore" (HOYA Technosurgical Corporation), and CAP such as "Cytotrans (registered trademark) Granules (GC Corporation)."

The shape of the bone substitute material used in the complex of the present invention is not particularly limited. The shape of the bone substitute material can be selected appropriately depending on the form of bone regeneration required. For example, when regeneration of bone with a relatively complex shape is desired, one or more complexes of the present invention, in which granular bone substitute materials are covered with a layered cell sheet, can be used as necessary. When regeneration of bone with a relatively simple shape is desired, the complex of the present invention, in which a bone substitute material block made using a CAD/CAM (computer-aided design/computer-aided manufacturing) system to fit the site to be regenerated is covered with a layered cell sheet, can be used.

In one embodiment, the bone substitute material used in the complex of the present invention is characterized by its granular form. Granular bone substitute materials generally have a particle size of about 0.15 mm to 2 mm, and it is difficult to handle individual granules. However, the present invention is characterized by being granular, which allows for adjustment of the size of the complex to be produced, while covering with the layered cell sheet ensures operability. The particle size of the bone substitute material used to produce the complex of the present invention can be, but not limited to, generally 0.01 mm or more, preferably 0.1 mm or more, 0.2 mm or more, 0.3 mm or more, more preferably 0.5 mm or more. The particle size of the bone substitute material can be, but not limited to, generally 10 mm or less, preferably 6 mm or less, 5 mm or less, 4 mm or less, more preferably 3 mm or less. In one embodiment, the particle size of the bone substitute material can be, but not limited to, generally 0.01 to 10 mm, preferably 0.1 to 6 mm, 0.2 to 5 mm, 0.3 to 4 mm, more preferably 0.5 to 3 mm.

The complex of the present invention is characterized in that the bone substitute material is covered with a layered cell sheet containing osteogenic cells. In the present specification, the "osteogenic cell" refers to a cell directly involved in bone formation or a precursor cell therefor. Examples of osteogenic cells suitable for use in preparing the complex of the present invention include, but are not limited to, mesenchymal stem cells, osteoblasts, and bone marrow-derived stromal cells. The tissue from which mesenchymal stem cells are derived may be any tissue, such as bone marrow, adipose tissue, umbilical cord, placenta, dental pulp, or synovium. The osteogenic cells may also be derived from iPS cells.

In the present specification, the "layered cell sheet" means a structure composed of a large number of cells layered together and an extracellular matrix derived from those cells. A layered cell sheet differs from a monolayer cell sheet in that at least some of the cells are biologically bonded in three dimensions. A layered cell sheet with such biological bonds is characterized by the fact that the bonds between the cells are not substantially destroyed, and as a result, it can be handled as a sheet. Such biological bonds include bonds between cells via the extracellular matrix. Such a layered cell sheet does not refer to a construct obtained merely by stacking single-layer cell sheets, but rather refers to a sheet formed such that the cells constitute a multi-layer structure of two or more layers at the time of cell sheet formation. The number of cells contained in the layered cell sheet of the present invention can be 3×10⁶ to 10×10⁶ cells, 4×10⁶ to 8×10⁶ cells, 5×10⁶ to 6×10⁶ cells, each per 9.5 cm², 8×10⁶ to 14×10⁶ cells, 10×10⁶ to 12×10⁶ cells, 11×10⁶ cells, each per 21 cm², 22×10⁶ to 42×10⁶ cells, 27×10⁶ to 37×10⁶ cells, 32×10⁶ cells, each per 55 cm². Alternatively, the number of cells per unit area (cm²) of the culture vessel can be 3×10⁵ to 2×10⁶ cells, 3×10⁵ to 1×10⁶ cells, 3×10⁵ to 7×10⁵ cells, 4×10⁵ to 8×10⁵ cells, 5×10⁵ to 7×10⁵ cells, 5×10⁵ to 6×10⁵ cells, 5×10⁵ cells, or 6×10⁵ cells.

A layered cell sheet containing osteogenic cells can be prepared by a known method (e.g., Ando, W. et al., Tissue Eng Part A. 2008; 14(12):2041-9.). Briefly, osteogenic cells are seeded in a cell culture vessel at about 20 times to 160 times the general seeding amount, and adherent culture is performed in a medium and a temperature and humidity environment appropriate for the cells, for example, in a humidified incubator at 37°C, 5% CO₂, until a multilayer structure is formed. In one embodiment, cells are seeded at 1.0×10⁶ to 8×10⁶ cells, preferably 1.8×10⁶ to 7.2×10⁶ cells, per 35 mm dish (9.5 cm²). Alternatively, cells are seeded at 1.0×10⁵ to 8×10⁵ cells/cm², preferably 2×10⁵ to 8×10⁵ cells/cm², per unit area of the culture vessel. As the culture medium, α-MEM containing 10% FBS, 1% antibiotic-antimycotic, and 0.2 mM ascorbic acid is used and a layered cell sheet can be prepared by culturing the cells for 10 days or more, e.g., 10 to 20 days, or 10 to 14 days.

In preparing the complex of the present invention, the method for covering the bone substitute material with a layered cell sheet containing osteogenic cells is not particularly limited as long as it can cover the bone substitute material with the layered cell sheet. Typically, the bone substitute material is placed in the center of the layered cell sheet. The edge of the layered cell sheet is then grasped using tweezers or the like, and the layered cell sheet is moved so that the bone substitute material will be enveloped by the layered cell sheet, whereby the bone substitute material is covered with the layered cell sheet. If necessary, the method may include a step of first performing adherent culture of osteogenic cells in a culture dish to prepare a layered cell sheet. The layered cell sheet does not need to cover 100% of the surface of the bone filling material as long as the object of the present invention can be achieved. For example, the coverage is sufficient as long as the complex can be held when grasped with tweezers or a similar grasping tool.

The shape of the complex of the present invention is not particularly limited as long as it is a shape that fits the bone defect site. The shape of the complex of the present invention may be, for example, substantially spherical, substantially cylindrical, substantially rectangular, or the like, and in one preferred embodiment, the shape of the complex of the present invention is substantially spherical. By being substantially spherical, the complex of the present invention can be expected to show the same effect regardless of the direction from which it is implanted into a living body. Furthermore, because the direction of implantation is not restricted, any part of the complex can be grasped with tweezers, and the handling of the complex becomes advantageously easy. The method for forming the complex into an substantially spherical shape is not particularly limited. The complex may be formed into an substantially spherical shape when the bone substitute material is covered with the layered cell sheet using tweezers or the like, or the bone substitute material may be covered with the layered cell sheet to prepare the complex, and then the complex may be formed into a substantially spherical shape using tweezers or the like. In the present specification, "substantially spherical" refers to a shape that can be recognized as a sphere. Specifically, "substantially spherical" includes both perfect spheres and shapes that are not perfect spheres but are recognizable as spheres.

The complex of the present invention can be prepared in various sizes by adjusting the amount of bone substitute material and the area of the layered cell sheet. When the complex of the present invention is substantially spherical, its size may be, but not limited to, generally major axis 1 to 30 mm, minor axis 1 to 20 mm, major axis 2 to 25 mm, minor axis 1 to 15 mm, major axis 3 to 20 mm, minor axis 1 to 10 mm, major axis 4 to 15 mm, minor axis 2 to 8 mm, major axis 6 to 10 mm, minor axis 4 to 6 mm, major axis 1 to 10 mm, minor axis 1 to 10 mm, preferably major axis 1 to 9 mm, minor axis 1 to 9 mm, major axis 1 to 8 mm, minor axis 1 to 8 mm, major axis 1 to 7 mm, minor axis 1 to 7 mm, more preferably major axis 1 to 6 mm, minor axis 1 to 6 mm. In the present specification, the "major axis" and "minor axis" refer to the longest diameter and the shortest diameter of the substantial sphere. The complex of the present invention can be prepared by covering one or more block-shaped bone substitute materials with a layered cell sheet. The size thereof can be, but is not limited to, generally 10 to 100 mm in length, 10 to 40 mm in width, and 5 to 20 mm in height.

In one preferred embodiment of the present invention, the complex of the present invention contains granular bone substitute materials covered with a layered cell sheet. The complex is substantially spherical, with a major axis of 3 to 10 mm, or 3 to 5 mm, and a minor axis of 3 to 6 mm, or 3 to 5 mm, and the number of osteogenic cells present on the surface of the complex can be 1.0×10⁶ to 6.0×10⁶ cells. A complex of this size shows good operability, allowing easy grasping with tweezers, can be easily implanted into relatively narrow parts such as the oral cavity, and enables horizontal and vertical bone regeneration and/or augmentation. These sizes are therefore preferred for implementing the present invention. When preparing a complex of this size, a layered cell sheet having a diameter of about 25 to 90 mm or 25 to 50 mm (e.g., diameter 35 mm) covers about 15 to 25 mg (e.g., 20 mg) of bone substitute materials. The amount of the bone substitute materials per area (cm²) of the layered cell sheet can be 1 to 4 g/cm², 2 to 3 g/cm², 2 g/cm². The amount of the bone substitute material per cell number (1×10⁶ cells) can be 2 to 5 mg/1×10⁶ cells, 2.5 to 4 mg/1×10⁶ cells, 3 mg/1×10⁶ cells. Furthermore, by appropriately adjusting the cell number at seeding, culture time, culture temperature, and the like, a layered cell sheet containing a desired number of cells can be easily produced by those of ordinary skill in the art. Regarding the determination of the number of osteogenic cells present on the surface of the complex, when the suitable cell number is confirmed in an experiment using rats (i.e., cells:rat osteogenic cells/implantation target:rat), the suitable cell number in human can be obtained by multiplying the suitable cell number in rats by 0.67

### 2. Production method of complex for bone regeneration or augmentation

The present invention also provides a method for producing a complex for bone regeneration or augmentation, including the following steps (hereinafter sometimes referred to as "the production method of the present invention"):
a step of covering bone substitute material with a layered cell sheet containing osteogenic cells.

In one embodiment, the production method of the present invention may further include a step of preparing a layered cell sheet containing osteogenic cells before the step of covering bone substitute material with a layered cell sheet containing osteogenic cells.

The layered cell sheet containing osteogenic cells, method for preparing the same, bone substitute material, and the like in the production method of the present invention are the same as those described for the complex of the present invention.

In one embodiment, the production method of the present invention may further include a step of forming the complex into a substantially spherical shape, simultaneously with or after the step of covering the bone substitute material with the layered cell sheet containing osteogenic cells. The effects obtained by forming the complex into a substantially spherical shape and the formation method are also as described above. The preferred size of the substantially spherical complex and the number of osteogenic cells present on its surface are also as described above.

### 3. Kit for producing complex for bone regeneration or augmentation

The present invention also provides a kit for producing a complex for bone regeneration or augmentation, containing bone substitute material and a layered cell sheet containing osteogenic cells (hereinafter sometimes referred to as "the kit of the present invention").

The bone substitute material, layered cell sheet containing osteogenic cells, and the like in the kit of the present invention are the same as those described for the complex of the present invention.

The kit of the present invention may also contain constituent elements other than the bone substitute material and the layered cell sheet containing osteogenic cells. Examples of such constituent elements include, but are not limited to, sterilized tweezers and instruction manual.

### 4. Layered cell sheet containing osteogenic cells, which is for use in producing complex for bone regeneration or augmentation

The present invention also provides a layered cell sheet containing osteogenic cells, which is for use in producing a complex for bone regeneration or augmentation by combining with bone substitute material (hereinafter sometimes referred to as "the layered cell sheet of the present invention").

The layered cell sheet containing osteogenic cells, method for preparing the same, bone substitute material, and the like in the layered cell sheet of the present invention are the same as those described for the complex of the present invention.

This embodiment can also be rephrased as "a layered cell sheet composition for producing a complex for bone regeneration or augmentation, characterized in that the complex for bone regeneration or augmentation is produced by covering bone substitute material with a layered cell sheet composition containing osteogenic cells." Alternatively, this embodiment can also be rephrased as "use of a layered cell sheet containing osteogenic cells in the production of a complex for bone regeneration or augmentation, characterized in that the aforementioned complex for bone regeneration or augmentation is produced by covering bone substitute material with the aforementioned layered cell sheet containing osteogenic cells."

### 5. Bone substitute material for use in producing complex for bone regeneration or augmentation

The present invention also provides bone substitute material for use in producing a complex for bone regeneration or augmentation by combining with a layered cell sheet containing osteogenic cells (hereinafter sometimes referred to as "the bone substitute material of the present invention").

The bone substitute material, layered cell sheet containing osteogenic cells, method for preparing the same, and the like in the bone substitute material of the present invention are the same as those described for the complex of the present invention.

This embodiment can also be rephrased as "a bone substitute material composition for producing a complex for bone regeneration or augmentation, characterized in that the complex for bone regeneration or augmentation is produced by covering the bone substitute material with a layered cell sheet containing osteogenic cells." Alternatively, this embodiment can also be rephrased as "use of a bone substitute material in the production of a complex for bone regeneration or augmentation, characterized in that the aforementioned complex for bone regeneration or augmentation is produced by covering the aforementioned bone substitute material with the layered cell sheet containing osteogenic cells."

### 6. Method for treating bone defect

The present invention also provides a method for treating a bone defect in a subject, including a step of implanting the complex of the present invention into a subject having a bone defect (hereinafter referred to as "the treatment method of the present invention").

In the treatment method of the present invention, the step of implanting the complex of the present invention into a subject having a bone defect may be performed by a method known per se. Briefly, a surgical procedure is used to expose a bone defect site or its vicinity (in other words, the site where bone regeneration or augmentation is intended) in a subject, and the complex of the present invention can be implanted into the bone defect site or its vicinity by placing the complex of the present invention at the bone defect site or its vicinity by using tweezers or the like. During implantation, only one complex of the present invention may be placed at the bone defect site or its vicinity, or multiple complexes can also be placed as necessary.

The present invention is described in more detail in the following Examples, but the present invention is not limited to those Examples in any way.

### [Example]

### [Example 1] Implantation of complex of the present invention prepared using rat femur-derived bone marrow mesenchymal stem cells into parietal region of rat

### [Cell culture of osteogenic cells and preparation of layered cell sheet]

Bone marrow was harvested from the femur of a 6-week-old male F344 rat (see Ando, W. et al., Tissue Eng Part A. 2008; 14(12):2041-9). The harvested bone marrow was seeded in a 90 mm dish (Corning), and the expanded cells were used as bone marrow mesenchymal stem cells (hereinafter sometimes referred to as "MSCs"). The medium used was α-MEM (Thermo Fisher Scientific) containing 10% FBS and 1% antibiotic-antimycotic (Thermo Fisher Scientific). MSCs were cultured in a humidified incubator at 37°C, 5% CO₂. The medium was changed every three days. Cells were passaged using trypsin EDTA (0.25%, Thermo Fisher Scientific) before reaching 100% confluence. At the second passage, cells were seeded in 35 mm dishes at 5.4×10⁶ cells/dish. After seeding into the 35 mm dishes, the medium used was α-MEM containing 10% FBS, 1% antibiotic-antimycotic, and 0.2 mM ascorbic acid. The medium was changed daily and the cells were cultured for 14 days to prepare layered cell sheets. The prepared layered cell sheets could be detached from the dishes without using any additional enzymatic treatment or equipment. The layered cell sheets composed of cells that were layered, that could be grasped with tweezers, and were strong enough to maintain a substantially spherical shape even when used to cover bone substitute material were used.

### [Preparation of complex of the present invention]

The complex of the present invention was prepared by covering 20 mg of β-TCP (OSFERION^{®}, Olympus Corporation) as bone substitute material, with a layered cell sheet. The prepared complex of the present invention was substantially spherical and had a diameter of about 4 mm. The prepared complex was implanted under the periosteum of the parietal bone of F344 rats (the gray circular area in Fig. 2(a)).

### [Implantation experiment]

Three 6-week-old male F344 rats (KBT Oriental, Ltd.) were used. The rats were anesthetized with inhalation anesthesia using isoflurane (1%-2%) and local anesthesia using xylocaine (0.2%, 0.1 ml). After anesthesia, the animals were cleaned with povidone-iodine, an about 1 cm wide incision was made, including the periosteum, and the periosteum was peeled off to expose the parietal bone. Then, the complex of the present invention was implanted under the periosteum, and the skin and periosteum were sutured with absorbable sutures. During surgery, xylocaine (2%, 0.1 ml) was administered locally to reduce postoperative pain. Furthermore, 6 mg/kg of gentamicin was administered intraperitoneally to prevent postoperative infection. After 12 weeks, the rats were euthanized, and the implantation site was collected and subjected to HE staining. The results are shown in Fig. 2(b).

As shown in Fig. 2(b), vertical bone augmentation could be achieved by implanting the complex of the present invention into the parietal region of the rat. Furthermore, formation of new bone was confirmed not only from the host bone side but also from the center of the complex of the present invention. In general, application of bone substitute material to an implant site is a relatively difficult process due to the small particle size of the bone substitute material. However, the use of the complex of the present invention extremely simplified the implantation process.

### [Example 2] Implantation of complex of the present invention, prepared using human iliac bone marrow mesenchymal stem cells, into immunodeficient mouse

### [Cells]

Three strains of human iliac bone marrow mesenchymal stem cells (MSCs) were purchased from Lonza. The medium used was α-MEM (Thermo Fisher Scientific) containing 10% FBS and 1% antibiotic-antimycotic (Thermo Fisher Scientific). MSCs were seeded in 90 mm dishes and cultured in a humidified incubator at 37°C, 5% CO₂. The medium was changed every three days. Cells were passaged using trypsin EDTA (0.25%, Thermo Fisher Scientific) before reaching 100% confluence. For the 3rd to 6th passages, cells were seeded in 35 mm dishes at 3.6×10⁶ cells/dish. After seeding into the 35 mm dishes, the medium used was α-MEM containing 10% FBS, 1% antibiotic-antimycotic, and 0.2 mM ascorbic acid. The medium was changed daily and the cells were cultured for 7 days to prepare layered cell sheets.

### [Preparation of complex of the present invention]

50 mg of carbonate apatite (Cytrans^{®} Granules, Fordi Co., Ltd.) was covered with a layered cell sheet of each of the three cell lines to prepare the complex of the present invention. As a control, β-TCP was mixed with 3% atelocollagen (Koken Co., Ltd.) and incubated at 37°C for 30 min for gelation to prepare a complex. These four complexes were implanted under the periosteum of the parietal bone of C. B-17 SCID mice.

### [Implantation experiment]

Twelve 4-week-old male C. B-17 SCID mice (KBT Oriental Co., Ltd.) were used. The mice were anesthetized with inhalation anesthesia using isoflurane (1%-2%) and local anesthesia using xylocaine (0.2%, 0.1 ml). After anesthesia, the animals were cleaned with povidone-iodine, an about 1 cm wide incision was made, including the periosteum, and the periosteum was peeled off to expose the parietal bone. Then, each of the four complexes was implanted under the periosteum of three mice, and the skin and periosteum were sutured with absorbable sutures. During surgery, xylocaine (2%, 0.1 ml) was administered locally to reduce postoperative pain. Furthermore, 6 mg/kg of gentamicin was administered intraperitoneally to prevent postoperative infection. After 12 weeks, the mice were euthanized, and the implantation site was collected and subjected to HE staining and human-specific Vimentin immunostaining. The results are shown in Fig. 3.

As shown in Fig. 3(a), bone augmentation was observed also in the complex of the present invention prepared using human-derived mesenchymal stem cells. In addition, immunostaining for human-specific Vimentin showed that cells present on the surface of the complex of the present invention are directly involved in bone formation, and that some of these cells migrated to the center of the complex after implantation. It has been reported that implanted cells cannot remain in the local area and become undetectable over time (Zimmermann, C.E. et al., Tissue Eng Part A. 2011; 17(7-9):1147-56). However, this Example shows that the implanted cells not only survived 12 weeks after implantation, but also differentiated into osteocytes. This is considered to be because an environment permitting cell survival is maintained by the layered cells and extracellular matrix derived from the cells, and cytokines secreted by the surviving cells exhibits the paracrine/autocrine effect. Furthermore, as in Example 1, formation of new bone was confirmed not only from the host bone side but also from the center of the complex of the present invention. Furthermore, compared to the control, the complex of the present invention showed an increased rate of new bone by about two-fold or more, demonstrating that the complex of the present invention promotes the formation of new bone extremely efficiently (Fig. 3(b)).

### [Example 3] Examination of size of complex of the present invention

### [Cells]

Bone marrow was harvested from the femur of 6-week-old male F344 rats. The harvested bone marrow was seeded in 90 mm dishes (Corning), and the expanded cells were used as MSCs. The medium used was α-MEM (Thermo Fisher Scientific) containing 10% FBS and 1% antibiotic-antimycotic (Thermo Fisher Scientific). MSCs were cultured in a humidified incubator at 37°C, 5% CO₂, and the medium was changed every three days. Cells were passaged using trypsin EDTA (0.25%, Thermo Fisher Scientific) before reaching 100% confluence. For the second passage, the cells were each seeded in 35 mm dish, 60 mm dish, or 90 mm dish. The cells were seeded in 35 mm dish at 5.4×10⁶ cells/dish, in 60 mm dish at 11.0×10⁶ cells/dish, and in 90 mm dish at 32.0×10⁶ cells/dish. After seeding, the medium used for each dish was α-MEM containing 10% FBS, 1% antibiotic-antimycotic, and 0.2 mM ascorbic acid. The medium was changed daily, and the cells were cultured for 14 days to prepare layered cell sheets.

### [Preparation of complex of the present invention]

The complexes with different sizes of the present invention were prepared by covering 20 mg of β-TCP (OSFERION^{®}) in 35 mm dish, 40 mg thereof in 60 mm dish, and 120 mg thereof in 90 mm dish with a layered cell sheet. The ratio of cell number to culture area and bone substitute material weight was standardized. The dish, dish area, cell number, and bone substitute material amount used are shown in Table 1. Fig. 4 shows the appearance of the prepared complexes with different sizes of the present invention. The complex prepared in the 35 mm dish was substantially spherical, with both the major axe and the minor axe of about 4 mm; the complex prepared in the 60 mm dish was substantially spherical, with both the major axe and the minor axe of about 6 mm; and the complex prepared in the 90 mm dish was substantially spherical, with the major axe of about 10 mm and the minor axe of about 6 mm. These complexes were implanted under the periosteum of the parietal bone of F344 rats.

**[Table 1]**

| dish (diameter) | dish (area) | cell number | bone substitute material (weight) |
|---|---|---|---|
| 35 mm | 9.5 cm² | 5.4×10⁶ | 20 mg |
| 60 mm | 21.0 cm² | 11.0×10⁶ | 40 mg |
| 90 mm | 55.0 cm² | 32.0×10⁶ | 120 mg |

### [Implantation experiment]

Three 6-week-old male F344 rats (KBT Oriental, Ltd.) were used. The rats were anesthetized with inhalation anesthesia using isoflurane (1%-2%) and local anesthesia using xylocaine (0.2%, 0.1 ml). After anesthesia, the animals were cleaned with povidone-iodine, an about 1 cm wide incision was made, including the periosteum, and the periosteum was peeled off to expose the parietal bone. Then, the complex prepared in each dish was implanted under the periosteum (N=1), and the skin and periosteum were sutured with absorbable sutures. During surgery, xylocaine (2%, 0.1 ml) was administered locally to reduce postoperative pain. Furthermore, 6 mg/kg of gentamicin was administered intraperitoneally to prevent postoperative infection. After 12 weeks, the rats were euthanized, and the implantation site was collected and subjected to HE staining. The results are shown in Fig. 5.

As shown in Fig. 5, good bone augmentation was confirmed even with larger-sized complexes of the present invention. Even when larger-sized complexes of the present invention were implanted, bone formation was confirmed on the host bone side, the center of the complex, and the surface layer side. In rats implanted with the complex of the present invention prepared in a 90 mm dish, formation of bone marrow-like tissue was confirmed (two arrow parts in Fig. 5). The larger the size of the complex, the greater the bone augmentation achieved with ease.

### [Example 4] Implantation of complex with different size of the present invention under condition of standardized cell number

### [Cells]

Bone marrow was harvested from the femur of 6-week-old male F344 rats. The harvested bone marrow was seeded in 90 mm dishes (Corning), and the expanded cells were used as MSCs. The medium used was α-MEM (Thermo Fisher Scientific) containing 10% FBS and 1% antibiotic-antimycotic (Thermo Fisher Scientific). MSCs were cultured in a humidified incubator at 37°C, 5% CO₂, and the medium was changed every three days. Cells were passaged using trypsin EDTA (0.25%, Thermo Fisher Scientific) before reaching 100% confluence. For the second passage, the cells were each passaged in 35 mm dish or 90 mm dish. The cells were seeded in 35 mm dish at 5.4×10⁶ cells/dish and in 90 mm dish at 32.0×10⁶ cells/dish. After seeding, the medium used for each dish was α-MEM containing 10% FBS, 1% antibiotic-antimycotic, and 0.2 mM ascorbic acid. The medium was changed daily, and the cells were cultured for 14 days to prepare layered cell sheets.

### [Preparation of complex of the present invention]

The complexes of the present invention were prepared by covering 20 mg of β-TCP (OSFERION^{®}) in 35 mm dish and 120 mg thereof in 90 mm dish with a layered cell sheet. These complexes were implanted under the periosteum of the parietal bone of F344 rats.

### [Implantation experiment]

Four 6-week-old male F344 rats (KBT Oriental, Ltd.) were used. The rats were anesthetized with inhalation anesthesia using isoflurane (1%-2%) and local anesthesia using xylocaine (0.2%, 0.1 ml). After anesthesia, the animals were cleaned with povidone-iodine, an about 1 cm incision was made down to the periosteum, and the periosteum was peeled off to expose the parietal bone. Then, the complex prepared in each dish was implanted under the periosteum. One complex prepared in the 90 mm dish was implanted into one rat, and six complexes prepared in the 35 mm dish were implanted into one rat, for a total of three rats. The diameter of the dish used, the number of complexes of the present invention implanted, and the total number of cells implanted into the rat are shown in Table 2. After implantation, the skin and periosteum were sutured with absorbable sutures. During surgery, xylocaine (2%, 0.1 ml) was administered locally to reduce postoperative pain. Furthermore, 6 mg/kg of gentamicin was administered intraperitoneally to prevent postoperative infection. After 12 weeks, the rats were euthanized, and the implantation site was collected and subjected to HE staining. The results are shown in Fig. 6.

**[Table 2]**

| dish diameter (area) | number of implanted complex of the present invention | total number of implanted cells |
|---|---|---|
| 35 mm (9.5 cm²) | 6 | 5.4×10⁶×6 |
| 90 mm (55.0 cm²) | 1 | 32.0×10⁶×1 |

As shown in Fig. 6, good bone augmentation was confirmed for the complex of the present invention with any size. When six complexes of the present invention prepared in a 35 mm dish were implanted, more favorable vertical bone augmentation could be achieved compared to when one complex of the present invention prepared in a 90 mm dish was implanted. In addition, the complex of the present invention prepared in a 35 mm dish was also more preferable than the complex of the present invention prepared in a 90 mm dish, from the viewpoint of operability.

### [Example 5] Examination of number of cells and culture days required for preparation of layered cell sheet

Bone marrow was harvested from the femur of 6-week-old male F344 rats. The harvested bone marrow was seeded in 90 mm dishes (Corning), and the expanded cells were used as MSCs. The medium used was α-MEM (Thermo Fisher Scientific) containing 10% FBS and 1% antibiotic-antimycotic (Thermo Fisher Scientific). MSCs were cultured in a humidified incubator at 37°C, 5% CO₂, and the medium was changed every three days. Cells were passaged using trypsin EDTA (0.25%, Thermo Fisher Scientific) before reaching 100% confluence. For the second passage, the cells were each passaged in 35 mm dish at 1.8×10⁶ cells/dish, 3.6×10⁶ cells/dish, 5.4×10⁶ cells/dish, or 7.2×10⁶ cells/dish, and the medium was changed daily. The cells were cultured for 14 days at maximum, and the number of culture days required for the preparation of layered cell sheets was examined. The results are shown in Table 3. The symbols in the Table mean the following:
×: unable to prepare layered cell sheet
Δ: possible to prepare layered cell sheet
○: possible to prepare good layered cell sheet

**[Table 3]**

| cell number | number of culture days | | | | |
|---|---|---|---|---|---|
| | 7 day | 10 day | 11 day | 12 day | 14 day |
| 1.8×10⁶ | × | × | × | x | ○ |
| 3.6×10⁶ | × | × | × | Δ | ○ |
| 5.4×10⁶ | × | × | Δ | ○ | ○ |
| 7.2×10⁶ | × | Δ | ○ | ○ | ○ |

As shown in Table 3, the number of culture days required to prepare a sheet varies depending on the number of cells prior to culture. However, it was shown that if about 1.0×10⁶ mesenchymal stem cells can be recovered from a living body, a good layered cell sheet (and the complex of the present invention) can be prepared within two weeks.

### [Industrial Applicability]

According to the present invention, it is possible to treat a wide range of bone defects requiring horizontal/vertical bone regeneration and/or bone augmentation. According to the present invention, moreover, it is possible to efficiently prepare a novel complex for bone regeneration or augmentation that enables such treatment. Therefore, the present invention is extremely beneficial in the orthopedic field and the dental field.

This application is based on a patent application No. 2023-102016 filed in Japan(filing date: June 21, 2023), the contents of which are incorporated in full herein. In addition, all references cited herein are incorporated by reference in their entirety into the present application.

## Claims

1. A complex for bone regeneration or augmentation, comprising bone substitute material covered with a layered cell sheet containing osteogenic cells.

2. The complex according to claim 1, wherein the bone substitute material is at least one selected from the group consisting of hydroxyapatite, β-tricalcium phosphate, and carbonate apatite.

3. The complex according to claim 1 or 2, wherein the osteogenic cell is at least one selected from the group consisting of a mesenchymal stem cell, an osteoblast, and a bone marrow-derived stromal cell.

4. A method for producing a complex for bone regeneration or augmentation, comprising the following step:
a step of covering bone substitute material with a layered cell sheet comprising osteogenic cells.

5. The production method according to claim 4, comprising a step of preparing the layered cell sheet comprising osteogenic cells before the step of covering bone substitute material with a layered cell sheet comprising osteogenic cells.

6. The production method according to claim 4 or 5, wherein the bone substitute material is at least one selected from the group consisting of hydroxyapatite, β-tricalcium phosphate, and carbonate apatite.

7. The production method according to claim 4 or 5, wherein the osteogenic cell is at least one selected from the group consisting of a mesenchymal stem cell, an osteoblast, and a bone marrow-derived stromal cell.

8. A kit for producing a complex for bone regeneration or augmentation, comprising bone substitute material and a layered cell sheet comprising osteogenic cells.

9. The kit according to claim 8, wherein the bone substitute material is at least one selected from the group consisting of hydroxyapatite, β-tricalcium phosphate, and carbonate apatite.

10. The kit according to claim 8 or 9, wherein the osteogenic cell is at least one selected from the group consisting of a mesenchymal stem cell, an osteoblast, and a bone marrow-derived stromal cell.

11. A layered cell sheet comprising osteogenic cells, which is for use in producing a complex for bone regeneration or augmentation by combining with bone substitute material.

12. The layered cell sheet according to claim 11, wherein the bone substitute material is at least one selected from the group consisting of hydroxyapatite, β-tricalcium phosphate, and carbonate apatite.

13. The layered cell sheet according to claim 11 or 12,
wherein the osteogenic cell is at least one selected from the group consisting of a mesenchymal stem cell, an osteoblast, and a bone marrow-derived stromal cell.

14. A bone substitute material, which is for use in producing a complex for bone regeneration or augmentation by combining with a layered cell sheet comprising osteogenic cells.

15. The bone substitute material according to claim 14,
wherein the bone substitute material is at least one selected from the group consisting of hydroxyapatite, β-tricalcium phosphate, and carbonate apatite.

16. The bone substitute material according to claim 14 or 15, wherein the osteogenic cell is at least one selected from the group consisting of a mesenchymal stem cell, an osteoblast, and a bone marrow-derived stromal cell.
